# EUROPEAN PATENT APPLICATION

(11) **EP 4 563 160 A1**
(43) Date of publication of application: **04.06.2025**
(21) Application number: 23846625.4
(22) Date of filing: 27.07.2023
(51) Int. Cl.: A61K 45/00, A61P 35/00, C12N 5/02, C12N 5/071, C12Q 1/04, A61K 31/444

(54) **TREATMENT AGENT FOR LUNG ADENOCARCINOMAS**

(30) Priority: 27.07.2022 JP 2022119657
(71) Applicant: Keio University, Tokyo, 108-8345 (JP)
(72) Inventor: YASUDA Hiroyuki, Tokyo 160-8582 (JP); SATO Toshiro, Tokyo 160-8582 (JP); EBISUDANI Toshiki, Tokyo 160-8582 (JP); FUKUNAGA Koichi, Tokyo 160-8582 (JP)
(74) Representative: Bryn Aarflot AS
(86) International application number: PCT/JP2023/027599
(87) International publication number: WO 2024/024895

(57) **Abstract**

Provided are: a therapeutic agent for NK2 homeobox 1 (NKX2-1)-negative lung adenocarcinoma containing a Porcupine inhibitor as an active component; a NKX2-1-negative lung adenocarcinoma organoid; and a method for screening for a therapeutic agent for NKX2-1-negative lung adenocarcinoma, the method including a step of culturing an NKX2-1-negative lung adenocarcinoma organoid in the presence of a test substance, and a step of measuring growth of the NKX2-1-negative lung adenocarcinoma organoid, wherein a decrease in the growth of the NKX2-1-negative lung adenocarcinoma organoid compared to a case in the absence of the test substance indicates that the test substance is a therapeutic agent for NKX2-1-negative lung adenocarcinoma.

## Description

### TECHNICAL FIELD

The present invention relates to a therapeutic agent for lung adenocarcinoma. More specifically, the present invention relates to an NKX2-1-negative lung adenocarcinoma organoid and a method for screening for a therapeutic agent for Wnt-dependent NKX2-1-negative lung adenocarcinoma, based on the ability to culture an organoid of a lung adenocarcinoma subgroup, which enabled production of an NKX2-1-negative lung adenocarcinoma organoid among lung adenocarcinomas and revealed that the NKX2-1-negative lung adenocarcinoma is Wnt-dependent. Priority is claimed on Japanese Patent Application No. 2022-119657, filed July 27, 2022, the content of which is incorporated herein by reference.

### BACKGROUND ART

Lung cancer is a disease with a poor prognosis that kills about 70,000 people a year in Japan. Lung adenocarcinoma is the most frequent type of lung cancer, accounting for just over 60% of all lung cancer cases in Japan.

Lung adenocarcinoma is thought to occur when cells derived from alveoli become cancerous. For this reason, it is known that the NKX2-1 (also called TTF1) gene, a transcription factor characteristic of alveoli, is highly expressed in lung adenocarcinoma as well as in alveoli. In these NKX2-1-expressing (NKX2-1-positive) typical lung adenocarcinomas, molecular-targeted therapeutic drugs targeting an EGFR gene mutation and an ALK fusion gene have been developed, which have made it possible to improve the prognosis of lung cancer patients with these genetic abnormalities.

However, it has been reported that there is NKX2-1-negative lung adenocarcinoma, which has few cases of EGFR gene mutations or ALK fusion gene positivity, and few patients can benefit from the molecular-targeted therapeutic drugs.

### Citation List

### Non Patent Documents

Non Patent Document 1: Sun, J.M., et al., Significance of Thymidylate Synthase and Thyroid Transcription Factor 1 Expression in Patients with Nonsquamous Non-small Cell Lung Cancer Treated with Pemetrexed-Based Chemotherapy, J Thorac Oncol. 6, 1392-1399, 2011
Non Patent Document 2: Kawasaki K., et al., An Organoid Biobank of Neuroendocrine Neoplasms Enables Genotype-Phenotype Mapping, Cell, 183, 1420-1435, 2020

### SUMMARY OF INVENTION

### Technical Problem

An object of the present invention is to analyze subgroups of lung adenocarcinoma and to provide a new therapeutic strategy for lung adenocarcinoma, particularly NKX2-1-negative lung adenocarcinoma.

### Solution to Problem

The present invention has the following aspects.
[1] A therapeutic agent for NK2 homeobox 1 (NKX2-1)-negative lung adenocarcinoma containing a Porcupine inhibitor as an active component.
[2] The therapeutic agent according to [1], in which the Porcupine inhibitor is C59 (CAS No. 1243243-89-1).
[3] An NKX2-1-negative lung adenocarcinoma organoid.
[4] A method for screening for a therapeutic agent for NKX2-1-negative lung adenocarcinoma, the method including: a step of culturing an NKX2-1-negative lung adenocarcinoma organoid in the presence of a test substance; and a step of measuring growth of the NKX2-1-negative lung adenocarcinoma organoid, wherein a decrease in the growth of the NKX2-1-negative lung adenocarcinoma organoid compared to a case in the absence of the test substance indicates that the test substance is a therapeutic agent for NKX2-1-negative lung adenocarcinoma.

### Advantageous Effects of Invention

According to the present invention, it is possible to provide a novel therapeutic strategy for lung adenocarcinoma, particularly NKX2-1-negative lung adenocarcinoma.

### BRIEF DESCRIPTION OF DRAWINGS

[FIG. 1A] Representative micrographs showing results of immunohistochemical staining of slice specimens of primary cancer tissues, lung cancer organoids, and xenografts in Experimental Example 2.
[FIG. 1B] Representative micrographs showing results of immunohistochemical staining of slice specimens of primary cancer tissues, lung cancer organoids, and xenografts in Experimental Example 2.
[FIG. 1C] Representative micrographs showing results of immunohistochemical staining of slice specimens of primary cancer tissues, lung cancer organoids, and xenografts in Experimental Example 2.
[FIG. 1D] Representative micrographs showing results of immunohistochemical staining of slice specimens of primary cancer tissues, lung cancer organoids, and xenografts in Experimental Example 2.
[FIG. 2] A view of a summary of analysis results of various histological subtypes (pathology/morphology), niche factor dependence, and genetic changes for lung cancer organoids of 43 strains in Experimental Example 3.
[FIG. 3A] A view showing a tSNE plot of transcriptomes of the lung cancer organoids analyzed in Experimental Example 3.
[FIG. 3B] A view showing a tSNE plot of methylomes of the lung cancer organoids analyzed in Experimental Example 3.
[FIG. 3C] A view showing a tSNE plot of ATAC-seq of the lung cancer organoids analyzed in Experimental Example 3.
[FIG. 4] A view of a summary of analysis results of histological subtypes (pathology/morphology), the expression level of HNF4A, the expression level of NKX2-1, Wnt dependence (Wnt-3A/R-spondin 1 (WR) dependence), morphology, and mutations of genes such as KRAS, EGFR, ERBB2, ALK, and BRAF for various lung adenocarcinoma organoid strains in Experimental Example 4.
[FIG. 5] Representative micrographs of Wnt-dependent lung adenocarcinoma organoid strains and Wnt-independent lung adenocarcinoma organoid strains cultured in the presence or absence of Wnt-3A/R-spondin 1 in Experimental Example 4.
[FIG. 6] Representative micrographs showing results of hematoxylin-eosin staining and NKX2-1 immunostaining of slice specimens of Wnt-dependent lung adenocarcinoma organoid strains, Wnt-independent lung adenocarcinoma organoid strains, and primary cancer tissue derived therefrom in Experimental Example 4.
[FIG. 7A] A schematic view illustrating an overview of Experimental Example 5.
[FIG. 7B] A view showing representative results of NKX2-1 expression confirmed by capillary-based immunoassay for wild-type and NKX2-1 knockout strains of Wnt-independent lung adenocarcinoma organoid strains in Experimental Example 5.
[FIG. 7C] Representative micrographs showing results of culture of the wild-type and NKX2-1 knockout strains of the Wnt-independent lung adenocarcinoma organoid strains in Experimental Example 5 in the presence or absence of Wnt-3A/R-spondin 1.
[FIG. 8A] A schematic view illustrating an overview of Experimental Example 5.
[FIG. 8B] A view showing representative results of NKX2-1 expression confirmed by capillary-based immunoassay for the Wnt-dependent lung adenocarcinoma organoid strains into which an NKX2-1 gene expression construct is introduced in Experimental Example 5 in the absence or presence of doxycycline.
[FIG. 8C] Representative micrographs showing results of culture of the Wnt-dependent organoid strains into which the NKX2-1 gene expression construct is introduced in Experimental Example 5 in the absence and presence of doxycycline and in the presence or absence of Wnt-3A/R-spondin 1.
[FIG. 9] Representative micrographs of Wnt-dependent lung adenocarcinoma organoid strains cultured in the absence of Wnt-3A (-Wnt), in the absence of Wnt-3A and in the presence of C59 (-Wnt+C59), and in the presence of Wnt-3A and C59 (+Wnt+C59) in Experimental Example 6.
[FIG. 10] Graphs showing results of quantitative RT-PCR measurement of expression levels of Wnt target genes LGR5 and AXIN2 in the Wnt-dependent lung adenocarcinoma organoid strains cultured in the absence of Wnt-3A (-Wnt), in the absence of Wnt-3A and in the presence of C59 (-Wnt+C59), and in the presence of Wnt-3A and C59 (+Wnt+C59) in Experimental Example 6.
[FIG. 11] A schematic view showing an experimental schedule of Experimental Example 7.
[FIG. 12] Representative photographs of kidneys removed from immunodeficient mice transplanted with lung adenocarcinoma organoids in Experimental Example 7, and fluorescence micrographs thereof.
[FIG. 13] Graphs showing measurement results of the tumor area in grafts in Experimental Example 7.
[FIG. 14] Schematic view showing a therapeutic strategy for lung adenocarcinoma.

### DESCRIPTION OF EMBODIMENTS

Wnt protein is a secretory glycoprotein with a molecular weight of about 40,000 and is conserved across species, from fruit flies to mammals. Wnt protein is known to be involved in regulation of early development, morphogenesis, cell proliferation and differentiation, and the like.

The Wnt signaling pathway includes the β-catenin pathway, which regulates gene expression via β-catenin, and the β-catenin-independent pathway, which is independent of the β-catenin pathway and regulates the cytoskeleton, cell motility, or the like.

As Wnt proteins, Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, Wntl6, and the like are identified in humans and mice.

In addition, as Wnt protein receptors, in addition to seven-pass transmembrane Frizzled (with 10 types known, Fz1 to Fz10), one-pass transmembrane types LRP5, LRP6, Ror2, and Ryk are also known.

Porcupine (PORCN) is an acyl transferase localized to the endoplasmic reticulum which performs palmitoylation of Wnt protein. All Wnt proteins are palmitoylated by Porcupine in the process of synthesis. NCBI accession numbers for mRNA of human Porcupine are NM_001282167.2, NM_022825.4, NM_203473.3, and the like. In addition, NCBI accession numbers for amino acid sequences of human Porcupine are NP_001269096.1, NP_073736.2, NP_982299.1, and the like.

The palmitoylated Wnt proteins bind to Evi/Wls, a seven-pass transmembrane sorting receptor, and are secreted extracellularly in a Wls dependent manner via the Golgi apparatus. Palmitoylation by Porcupine is thought to contribute not only to Wnt protein secretion, but also to binding to receptors and affinity for hydrophobic cell membranes.

### [Therapeutic agent for NKX2-1-negative lung adenocarcinoma]

In one embodiment, the present invention provides a therapeutic agent for NKX2-1-negative lung adenocarcinoma using a Porcupine inhibitor, a Wnt signal inhibitor, as an active component.

As will be described below in the examples, the present inventors have analyzed lung adenocarcinoma organoids in detail, and as a result, they have found for the first time that there is Wnt-dependent lung adenocarcinoma and Wnt-independent lung adenocarcinoma.

The fact that a cell is Wnt-dependent means that Wnt signaling is required for its growth. The fact that a cell is Wnt-independent means that it can grow without Wnt signaling.

As will be described below in the examples, the present inventors have further found for the first time that NKX2-1-negative lung adenocarcinoma is Wnt-dependent. The fact that a cell is NKX2-1 negative may mean that the expression level of NKX2-1 in that cell is significantly lower than in a control. Here, examples of controls include cells derived from alveoli. In addition, the expression level of NKX2-1 may be a value measured at an mRNA level or a value measured at a protein level. Alternatively, the fact that a cell is NKX2-1 negative may mean that the expression of NKX2-1 is not detected with the naked eye as a result of immunostaining of the cell.

In the therapeutic agent of the present embodiment, specific examples of Porcupine inhibitors include C59 (CAS No. 1243243-89-1), WNT974 (LGK974, CAS No. 1243244-14-5), ETC-1922159 (ETC-159, CAS No. 1638250-96-0), RXC004, and CGX1321.

The therapeutic agent of the present embodiment may be formulated as a pharmaceutical composition mixed with a pharmaceutically acceptable carrier. The pharmaceutical composition can be administered, for example, orally in the form of tablets, capsules, elixirs, microcapsules, and the like, or parenterally in the form of injections, suppositories, and the like.

As a pharmaceutically acceptable carrier, it is possible to use those normally used in preparations of pharmaceutical compositions. More specific examples thereof include: binders such as hypromellose, dextrin, macrogol 400, gelatin, corn starch, tragacanth gum, gum arabic; excipients such as lactose hydrate, D-mannitol, starch, crystalline cellulose, and alginic acid; and injection solvents such as water, ethanol, and glycerol.

The therapeutic drug of the present embodiment may include additives. Examples of additives include: lubricants such as calcium stearate and magnesium stearate; sweeteners such as sucrose, lactose, saccharin, and maltitol; flavoring agents such as peppermint and akamono oil; stabilizers such as carmellose sodium, hardened oil, light anhydrous silicic acid, povidone, glycerin fatty acid esters, benzyl alcohol, and phenol; buffer agents such as phosphates and sodium acetate; dissolution assistants such as benzyl benzoate and benzyl alcohol; and colorants such as yellow iron sesquioxide, iron sesquioxide, black iron oxide, and titanium oxide.

The pharmaceutical composition can be formulated by appropriately combining the above-described Porcupine inhibitor with the above-described pharmaceutically acceptable carrier and additive in a unit dosage form required for generally accepted pharmaceutical practice. In the pharmaceutical composition, the Porcupine inhibitor may be used alone or in combination of two or more thereof.

The appropriate daily dosage of the therapeutic drug or pharmaceutical composition is an amount containing an active component (Porcupine inhibitor) in the lowest effective dosage in view of producing a therapeutic effect. The lowest effective dosage depends on various factors including an activity of an active component contained in a pharmaceutical composition, functional group modification that defines liposolubility and water solubility, administration routes, administration time, discharge rate of a specific active component used, treatment periods, other drugs used in combination, age, sex, weight, diseases, health condition, a patient's pre-existing conditions, and other factors known in the medical art.

In general, the dosage of the therapeutic drug or pharmaceutical composition is an amount containing an active component (Porcupine inhibitor) in about 0.0001 to 100 mg/kg body weight per day. The therapeutic drug or pharmaceutical composition may be administered once a day or administered in approximately two to four divided doses per day.

### [NKX2-1-negative lung adenocarcinoma organoid]

In one embodiment, the present invention provides a Wnt-dependent lung adenocarcinoma organoid. An organoid is a three-dimensional cellular tissue body formed of cells accumulated and self-assembled, and has a structure and function similar to those of organs in a living body. "NKX2-1 negative" in an NKX2-1-negative lung adenocarcinoma organoid of the present embodiment is the same as described above. As will be described below in the examples, the present inventors were the first to find out that an NKX2-1-negative lung adenocarcinoma organoid is Wnt-dependent. The NKX2-1-negative lung adenocarcinoma organoid of the present embodiment is useful for analyzing the properties of NKX2-1 -negative lung adenocarcinoma, a subgroup of lung adenocarcinoma, and for screening for new therapeutic drugs for lung adenocarcinoma.

### [Method for screening for therapeutic agent for NKX2-1-negative lung adenocarcinoma]

In one embodiment, the present invention provides a method for screening for a therapeutic agent for NKX2-1-negative lung adenocarcinoma, the method including: a step of culturing an NKX2-1-negative lung adenocarcinoma organoid in the presence of a test substance; and a step of measuring growth of the NKX2-1-negative lung adenocarcinoma organoid, in which a decrease in the growth of the NKX2-1-negative lung adenocarcinoma organoid compared to a case in the absence of the test substance indicates that the test substance is a therapeutic agent for NKX2-1-negative lung adenocarcinoma.

In the screening method of the present embodiment, the test substance is not particularly limited, and, for example, a natural compound library, a synthetic compound library, an existing drug library, and the like can be used.

In the step of culturing an NKX2-1-negative lung adenocarcinoma organoid in the presence of a test substance, it is preferable to add a Wnt agonist to a medium.

The Wnt agonist means a drug that activates T-cell factor (hereinafter also referred to as TCF)/lymphoid enhancer factor (hereinafter referred to as LEF)-mediated transcription within cells. Accordingly, the Wnt agonist is not limited to a Wnt family protein, and includes a Wnt agonist that binds to and activates a Frizzled receptor family member, an inhibitor of intracellular β-catenin degradation, and an activator of TCF/LEF. The Wnt agonist is preferably at least one selected from the group consisting of Wnt protein, R-spondin, and a GSK-3β inhibitor.

A Wnt agonist more preferably include a complex of Wnt protein and afamin, a stabilizer thereof, and still more preferably include a complex of Wnt protein and afamin, and R-spondin.

The origin of Wnt protein is not particularly limited, and Wnt proteins derived from various organisms can be used. Among these, Wnt proteins derived from mammals are preferable. Examples of mammals include humans, mice, rats, cattle, pigs, and rabbits. Examples of mammalian Wnt proteins include those described above. Multiple types of Wnt proteins may be used in combination.

Examples of methods for preparing Wnt protein include a preparation method using Wnt protein-expressing cells. The origin (such as organism species and culture forms) of the Wnt protein-expressing cells is not particularly limited. Any cells that stably express Wnt protein may be used, and any cells that transiently express Wnt protein may be used. Examples of Wnt protein-expressing cells include L cells that stably express mouse Wnt3a (ATCC CRL-2647) and L cells that stably express mouse Wnt5a (ATCC CRL-2814). In addition, Wnt protein-expressing cells can be produced using a well-known gene recombination technique. In other words, by inserting DNA encoding desired Wnt protein into well-known expression vectors and introducing the resulting expression vectors into appropriate host cells, Wnt protein-expressing cells can be produced. Base sequences of genes encoding desired Wnt protein can be acquired from a well-known database such as GenBank, for example.

Wnt protein expressed by Wnt protein-expressing cells may be a fragment of the Wnt protein as long as it has Wnt activity, and may contain amino acid sequences other than the amino acid sequence of the Wnt protein. The amino acid sequences other than the amino acid sequence of the Wnt protein are not particularly limited, and examples thereof include amino acid sequences of affinity tags. In addition, it is unnecessary for the amino acid sequence of the Wnt protein to completely coincide with amino acid sequences that can be acquired from a well-known database such as GenBank, and may be substantially the same amino acid sequences as the amino acid sequences that can be acquired from the well-known database.

Examples of the amino acid sequences substantially the same as the amino acid sequence of the Wnt protein that can be acquired from the well-known database such as GenBank include an amino acid sequence in which one to several amino acids are deleted, substituted, or added in the amino acid sequences that can be acquired from the well-known database.

The amino acid sequence in which one to several amino acids are deleted, substituted, or added means an amino acid sequence in which a sufficient number of amino acids (10 or less is preferable, 7 or less is more preferable, and 6 or less is still more preferable) are deleted, substituted, or added to enable deletion, substitution, or addition through a well-known mutant peptide production method such as site-specific mutation induction method.

In addition, examples of substantially the same amino acid sequences include amino acid sequences having at least 80% or more, preferably at least 85% or more, more preferably at least 90% or more, still more preferably at least 92% or more, particularly preferably at least 95% or more, most preferably at least 99% or more identity to the amino acid sequences that can be acquired from the well-known database.

The concentration of the Wnt protein is preferably 50 ng/mL or more, more preferably 100 ng/mL to 10 µg/mL, still more preferably 200 ng/mL to 1 µg/mL, and particularly preferably 300 ng/mL to 1 µg/mL.

Examples of R-spondin include an R-spondin family consisting of R-spondin 1, R-spondin 2, R-spondin 3, and R-spondin 4. The R-spondin family is a secretory protein known to be involved in activation and regulation of Wnt signaling pathways. In a cell culture medium according to the present embodiment, multiple types of R-spondins may be used in combination.

The R-spondins may be fragments of R-spondin as long as they have R-spondin activity, and may contain amino acid sequences other than amino acid sequences of R-spondin.

Measurement of the growth of the NKX2-1-negative lung adenocarcinoma organoid can be performed through an appropriate method, for example, measurement based on microscopic observation and a cell proliferation assay with a tetrazolium compound such as MTT.

### [Other embodiments]

In one embodiment, the present invention provides a method for treating lung adenocarcinoma, the method including: a step of detecting expression of NKX2-1 in cancer cells derived from a patient with lung adenocarcinoma; and a step of administering an effective amount of a Porcupine inhibitor to the patient with lung adenocarcinoma when the expression level of the NKX2-1 is significantly lower than that of a control.

When the expression level of the NKX2-1 is significantly higher than that of a control, the patient may be given a molecular-targeted therapeutic drug selected based on a cancer-causing driver mutation. Examples of molecular-targeted therapeutic drugs include therapeutic drugs targeting EGFR gene mutation or ALK fusion genes.

Examples of molecular-targeted therapeutic drugs targeting EGFR include gefitinib, erlotinib, afatinib, osimertinib, dacomitinib, and necitumumab. In addition, examples of molecular-targeted therapeutic drugs targeting ALK fusion genes include alectinib, ceritinib, and lorlatinib.

In one embodiment, the present invention provides a Porcupine inhibitor for use in the treatment of lung adenocarcinoma with a significantly lower expression level of NKX2-1 than that of a control.

In one embodiment, the present invention provides use of a Porcupine inhibitor for producing a therapeutic drug for lung adenocarcinoma with a significantly lower expression level of NKX2-1 than that of a control.

In each of these embodiments, controls, the expression level of NKX2-1, and Porcupine inhibitors are the same as described above.

### Examples

Examples will be shown below to describe the present invention in more detail, but the present invention is not limited to the following examples.

### [Experimental Example 1]

### (Establishment of lung cancer organoid library)

This experiment was conducted using samples acquired from patients with written informed consent after approval from the Ethics Committee of Keio University School of Medicine. Cancer cells were collected from surgically resected samples, endoscopic biopsy samples, circulating tumor cells (CTCs), sputum, and pleural fluid derived from lung cancer patients.

Lung cancer organoids derived from a surgically resected sample, an endoscopic biopsy sample, sputum, and a pleural fluid sample were established in the same manner as in the method described in Kawasaki K., et al., An Organoid Biobank of Neuroendocrine Neoplasms Enables Genotype-Phenotype Mapping, Cell, 183 (5), 1420-1435, 2020.

Separation of CTCs was performed as follows. First, 15 mL of a blood sample was placed in a heparin tube. Subsequently, 750 µL of RosetteSep Human CD36 Depletion Cocktail (StemCell Technologies) was added to the blood and incubated for 20 minutes. Subsequently, red blood cells and white blood cells were removed by centrifugation for 20 minutes at 1200 × g using Ficoll-Paque PLUS (Cytiva), and CTCs were collected.

Subsequently, the collected CTCs were embedded in droplets of Matrigel (registered trademark) and overlaid and cultured in a complete medium. The complete medium was prepared by adding a niche factor with the composition shown in Table 2 below to a basic culture medium shown in Table 1 below. As a result, organoids were established from the CTCs.

**[Table 1]**

| Basic culture medium | |
|---|---|
| Component | Concentration |
| Advanced DMEM/F12 | - |
| HEPES | 10 mM |
| Penicillin-Streptomycin | 1% |
| GlutaMAX | 2 mM |
| B-27 supplement (Thermo Fisher Scientific Inc.) | 1 × |
| N-Acetyl-L-cyctein (FUJIFILM Wako Pure Chemical Corporation) | 1 mM |

**[Table 2]**

| Niche factor | |
|---|---|
| Component | Concentration |
| Afamin-Wnt-3A, Serum-free conditioned medium | 10% |
| Recombinant mouse EGF (Thermo Fisher Scientific Inc.) | 50 ng/mL |
| Recombinant human FGF-2 (PeproTech, Inc.) | 50 ng/mL |
| Recombinant human IGF-1 (BioLegent, Inc.) | 100 ng/mL |
| Recombinant human R-spondin 1 (R&D) | 5% |
| Recombinant mouse Noggin (PeproTech, Inc.) | 5% |
| A83-01 (Tocris Bioscience) | 500 nM |
| Y-27632 (FUJIFILM Wako Pure Chemical Corporation) | 10 µM |

Finally, organoids of 43 strains were established from cancer cells derived from lung cancer patients to obtain a lung cancer organoid library. As a result of immunohistochemical staining and microscopic observation, it was confirmed that lung adenocarcinoma (LUAD) organoids of 21 strains, squamous cell carcinoma (LUSC) organoids of 7 strains, small cell lung cancer (SCLC) organoids of 12 strains, large cell neuroendocrine carcinoma (LCNEC) organoids of 3 strains were obtained.

### [Experimental Example 2]

### (Analysis 1 of lung cancer organoid)

To verify preservation of pathological phenotypes of lung cancer organoids, lung cancer organoids were transplanted into immunodeficient mice to produce xenografts. Subsequently, pathological phenotypes thereof were compared to primary cancer tissues of original patients.

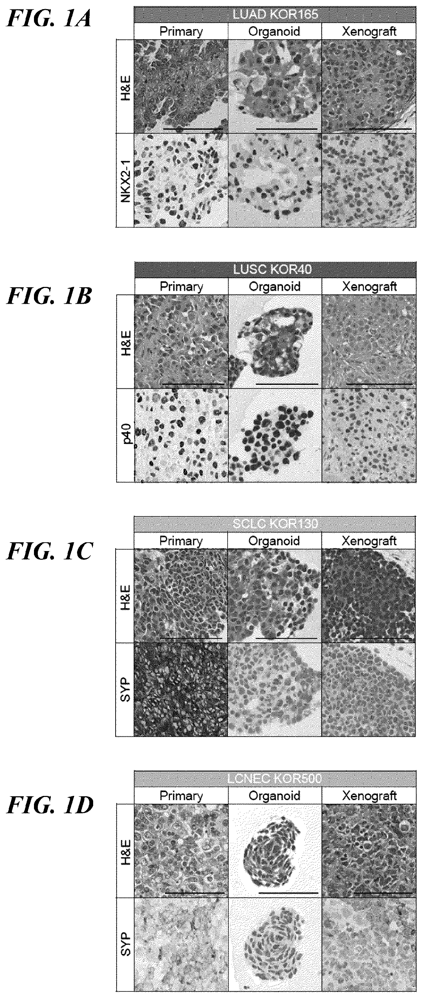

FIGS. 1A to 1D are representative micrographs showing results of immunohistochemical staining of slice specimens of primary cancer tissues, lung cancer organoids, and xenografts. FIG. 1A shows micrographs showing results of hematoxylin-eosin staining and NKX2-1 immunostaining of slice specimens of primary cancer tissue (left), lung cancer organoids (center), and xenografts (right) of lung adenocarcinoma (LUAD) organoid strains (KOR165 strains). NKX2-1 is a marker for lung adenocarcinoma.

FIG. 1B shows micrographs showing results of hematoxylin-eosin staining and p40 immunostaining of slice specimens of primary cancer tissues (left), lung cancer organoids (center), and xenografts (right) of squamous cell carcinoma (LUSC) organoid strains (KOR40 strains). p40 is a marker for squamous cell carcinoma.

FIG. 1C shows micrographs showing results of hematoxylin-eosin staining and synaptophysin (SYP) immunostaining of slice specimens of primary cancer tissues (left), lung cancer organoids (center), and xenografts (right) of small cell lung cancer (SCLC) organoid strains (KOR130 strain). The synaptophysin (SYP) is a marker for small cell lung cancer.

FIG. 1D shows micrographs showing results of hematoxylin-eosin staining and synaptophysin (SYP) immunostaining of slice specimens of primary cancer tissues (left), lung cancer organoids (center), and xenografts (right) of large cell neuroendocrine carcinoma (LCNEC) organoid strains (KOR500 strain). Synaptophysin (SYP) is a marker for neuroendocrine cells.

As a result, expression of pathological phenotypes and the histological subtype markers (NKX2-1, p40, and synaptophysin (SYP)) was consistent between the primary cancer tissues, the organoids, and the xenografts. These results showed that the resulting lung cancer organoid library is pathologically valid.

### [Experimental Example 3]

### (Analysis 2 of lung cancer organoid)

Niche factors were removed from lung cancer organoids to determine minimum requirements for the niche factors. If the organoids were able to survive for at least a month without the niche factors, the organoids were determined to be independent of the niche factors. If the organoids were able to survive but their growth declined after the removal of the niche factors, the niche factor dependence was determined by a survival rate after 3 months of culture.

**In** addition, whole-exome sequencing (WES) analysis and whole-genome sequencing (WGS) analysis were carried out to confirm genome abnormalities of the lung cancer organoids.

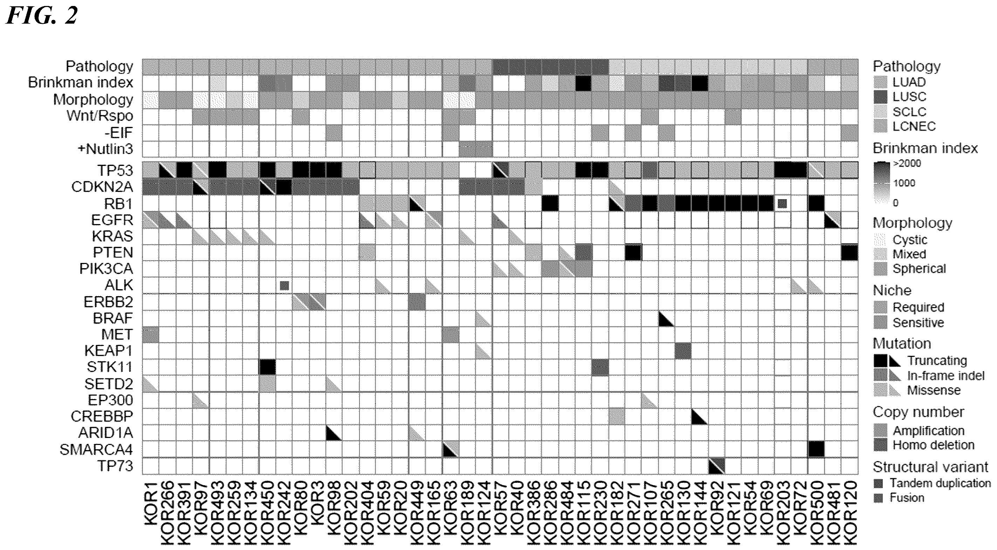

FIG. 2 is a view of a summary of analysis results of various histological subtypes (pathology/morphology), niche factor dependence, and genetic changes for lung cancer organoids of 43 strains.

The results showed that all the lung cancer organoids had TP53 mutations and/or EGFR/RAS signaling mutations, indicating that they originated from cancer cells. In addition, each lung cancer organoid showed genetic mutations, characteristic copy number changes, and aneuploidy consistent with the previously reported histological subtype patterns. For example, the EGFR/RAS signaling mutations were observed only in non-SCLC organoids. In addition, TP53 and RB1 mutations were frequently observed in SCLC and LCNEC.

Subsequently, RNA-seq, methylation microarray analysis, and assay for transposase-accessible chromatin with high-throughput sequencing (ATAC-seq) were performed, and transcriptomic and epigenetic profiling of lung cancer organoids were performed.

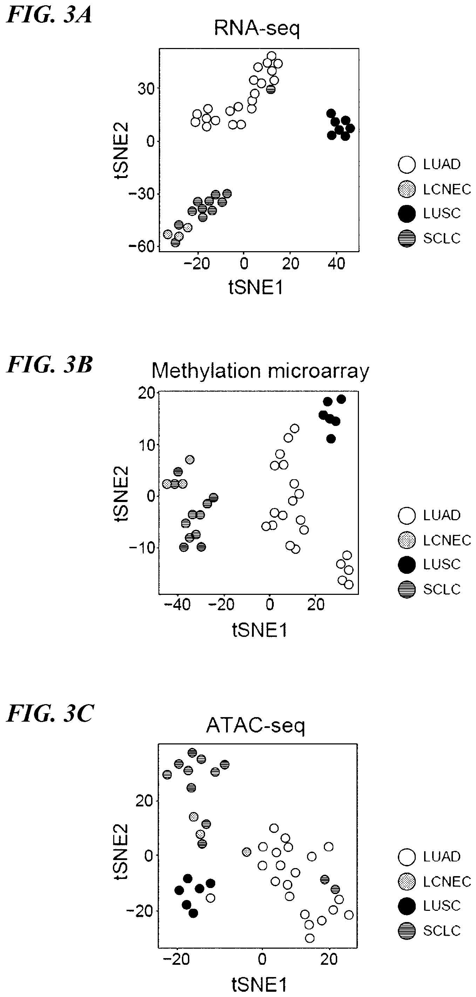

FIG. 3A is a view showing a tSNE plot of transcriptomes. Fig. 3B is a view showing a tSNE plot of methylomes. FIG. 3C is a view showing a tSNE plot of ATAC-seq. The tSNE analysis results of these multiomics data showed a population for each histological subtype of lung cancer organoids.

These results showed that the resulting lung cancer organoid library is valid at a molecular level.

### [Experimental Example 4]

### (Presence of Wnt-dependent subtype and Wnt-independent subtype in lung adenocarcinoma organoids)

As a result of examining niche factor dependence of lung cancer organoids, it was found that there are subtype showing different Wnt signaling dependencies in lung adenocarcinoma (LUAD) organoids.

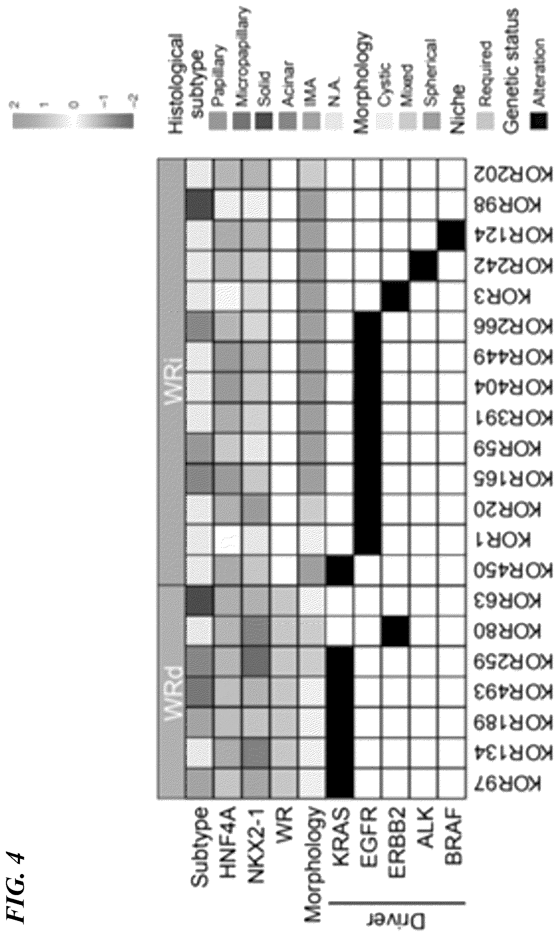

FIG. 4 is a view of a summary of analysis results of histological subtypes (pathology/morphology), the expression level of HNF4A, the expression level of NKX2-1, Wnt dependence (Wnt-3A/R-spondin 1 (WR) dependence), morphology, and mutations of genes such as KRAS, EGFR, ERBB2, ALK, and BRAF for various lung adenocarcinoma organoid strains. In FIG. 4, "WRd" indicates that it is Wnt-dependent, and "WRi" indicates that it is Wnt-independent.

The results revealed that one-third of the organoid strains derived from lung adenocarcinoma showed Wnt dependence, and that these organoids were NKX2-1 negative. In addition, it was revealed that the majority of the NKX2-1-negative lung adenocarcinoma organoids had KRAS mutations. It was revealed that the remaining lung adenocarcinoma organoid strains showed Wnt independence, and that most of them had EGFR mutations.

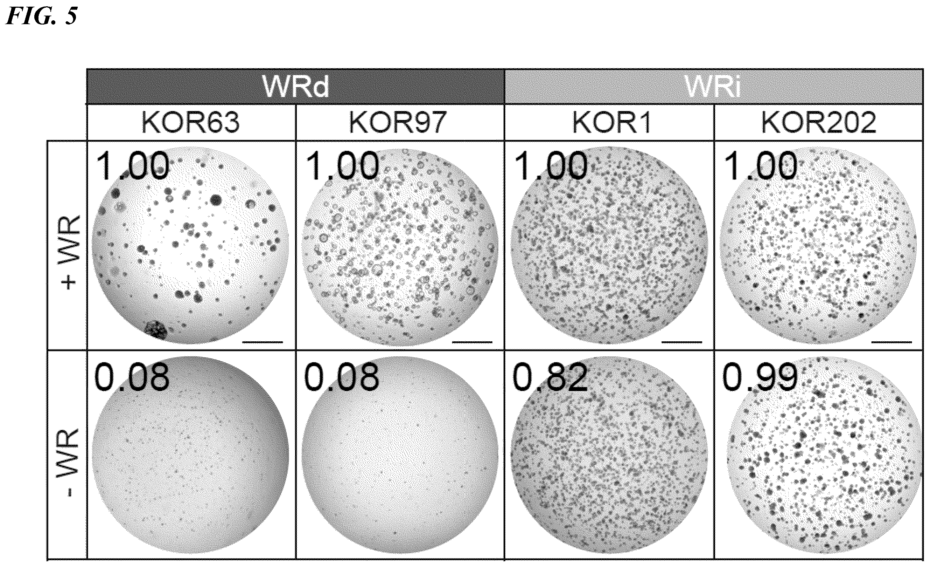

FIG. 5 shows representative micrographs of Wnt-dependent lung adenocarcinoma organoid strains (KOR63, KOR97) and Wnt-independent lung adenocarcinoma organoid strains (KOR1, KOR202) cultured in the presence or absence of Wnt-3A/R-spondin 1 (WR). The scale bar is 1 mm. In FIG. 5, "WRd" indicates that it is Wnt-dependent, and "WRi" indicates that it is Wnt-independent. In addition, "+WR" indicates a result of culturing in the presence of WR, and "-WR" indicates a result of culturing in the absence of WR. In addition, the number in the upper left corner of each image indicates the percentage of an area occupied by organoids cultured in the absence of WR when an area occupied by organoids cultured in the presence of WR is 1.

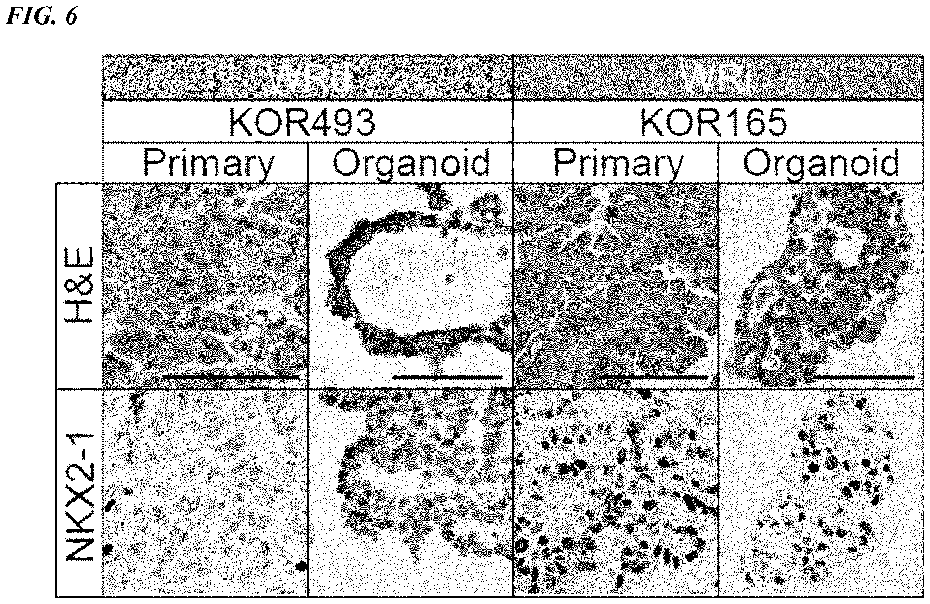

FIG. 6 shows representative micrographs showing results of hematoxylin-eosin staining and NKX2-1 immunostaining of slice specimens of Wnt-dependent lung adenocarcinoma organoid strains (KOR493), Wnt-independent lung adenocarcinoma organoid strains (KOR165), and primary cancer tissue derived therefrom. The scale bar is 100 µm.

The results revealed that the lung adenocarcinoma with reduced expression of NKX2-1 was Wnt-dependent. On the other hand, it was revealed that the Wnt-independent lung adenocarcinoma organoid strains expressed NKX2-1.

### [Experimental Example 5]

### (NKX2-1 determines Wnt dependence of LUAD)

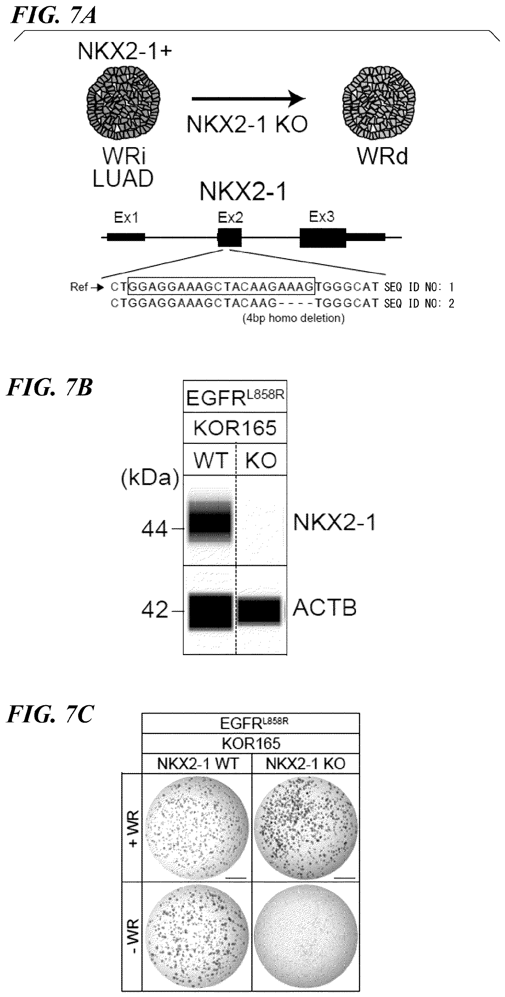

An NKX2-1 gene was knocked out by genome editing in three Wnt-independent lung adenocarcinoma organoid strains expressing NKX2-1. FIG. 7A is a schematic view illustrating an overview of this experiment. In FIG. 7A, "WRi" indicates that it is Wnt-independent, "WRd" indicates that it is Wnt-dependent, and "LUAD" indicates an lung adenocarcinoma organoid. The knockout of the NKX2-1 gene was confirmed by Sanger sequencing.

FIG. 7B is a view showing representative results of NKX2-1 expression confirmed by capillary-based immunoassay for wild-type and NKX2-1 knockout (KO) strains of Wnt-independent lung adenocarcinoma organoid strains (KOR165). Expression of ACTB was confirmed as a control. The result confirmed that the NKX2-1 knockout (KO) strains did not express NKX2-1.

FIG. 7C shows representative micrographs showing results of culture of the wild-type and NKX2-1 knockout (KO) strains of the Wnt-independent lung adenocarcinoma organoid strains (KOR165) in the presence or absence of Wnt-3A/R-spondin 1 (WR). In FIG. 7C, "+WR" indicates a result of culturing in the presence of WR, and "-WR" indicates a result of culturing in the absence of WR.

The results revealed that knocking out of the NKX2-1 gene in the Wnt-independent lung adenocarcinoma organoid strains resulted in a transition to Wnt dependence.

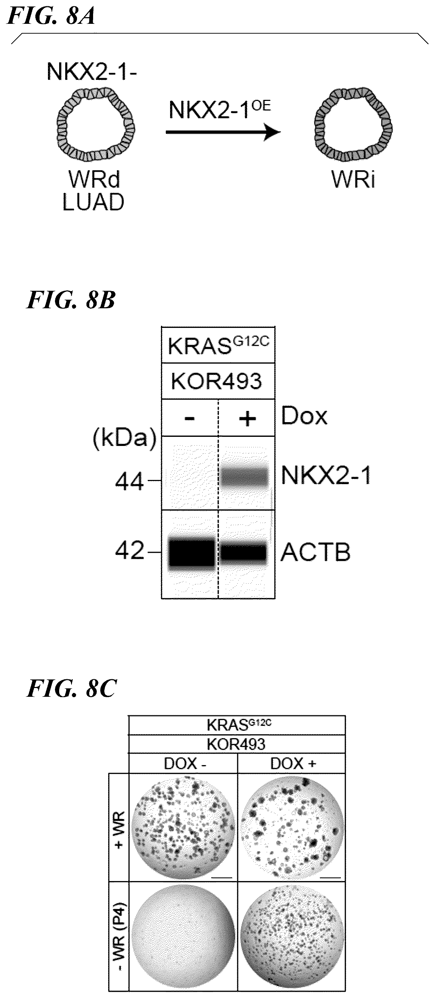

Subsequently, the NKX2-1 gene was overexpressed in three Wnt-dependent lung adenocarcinoma organoid strains that do not express NKX2-1. FIG. 8A is a schematic view illustrating an overview of this experiment. In this experiment, a construct enabling doxycycline (Dox)-dependent expression of the NKX2-1 gene was introduced.

FIG. 8B is a view showing representative results of NKX2-1 expression confirmed by capillary-based immunoassay for the Wnt-dependent lung adenocarcinoma organoid strains (KOR493) into which an NKX2-1 gene expression construct is introduced in the absence or presence of doxycycline. Expression of ACTB was confirmed as a control. In FIG. 8B "-" indicates the absence of doxycycline, and "+" indicates the presence of doxycycline. The result confirmed that the resulting organoid strains exhibited NKX2-1 in the presence of doxycycline.

FIG. 8C shows representative micrographs showing results of culture of the Wnt-dependent organoid strains (KOR165) into which the NKX2-1 gene expression construct is introduced in the absence and presence of doxycycline and in the presence or absence of Wnt-3A/R-spondin 1 (WR). In FIG. 8C "Dox-" indicates the absence of doxycycline, and "Dox+" indicates the presence of doxycycline. In addition, "+WR" indicates a result of culturing in the presence of WR, and "-WR" indicates a result of culturing in the absence of WR.

The results revealed that overexpression of the NKX2-1 gene in the Wnt-dependent organoid strains resulted in a transition to Wnt independence.

The above results revealed that expression of NKX2-1 determined the Wnt dependence of the lung adenocarcinoma organoids.

### [Experimental Example 6]

### (Examination 1 of therapeutic effect of NKX2-1-negative lung adenocarcinoma using Wnt-targetted therapy)

Porcupine (PORCN) is an acyl transferase localized to the endoplasmic reticulum which performs palmitoylation of Wnt protein. In this experimental example, the therapeutic effect of NKX2-1-negative lung adenocarcinoma was examined using C59 (CAS No. 1243243-89-1), a Porcupine inhibitor.

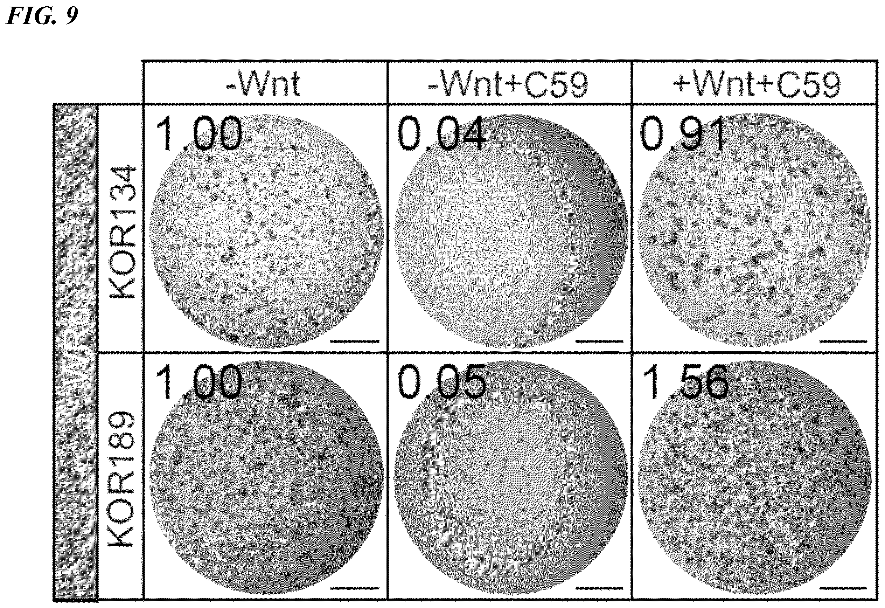

FIG. 9 shows representative micrographs of NKX2-1-negative lung adenocarcinoma organoid strains (KOR189, KOR134) cultured in the absence of Wnt-3A (-Wnt), in the absence of Wnt-3A and in the presence of C59 (-Wnt+C59), and in the presence of Wnt-3A and C59 (+Wnt+C59). The scale bar is 1 mm.

In FIG. 9, "WRd" indicates that it is Wnt-dependent. In addition, the number in the upper left corner of each image indicates the percentage of an area occupied by organoids cultured under each condition when an area occupied by organoids cultured in the absence of Wnt-3A is 1. KOR134 and KOR189 endogenously express Wnt protein but are Wnt-dependent, resulting in C59 growth inhibition, indicating that growth of Wnt-dependent NKX2-1-negative lung adenocarcinoma is inhibited by the Porcupine inhibitor.

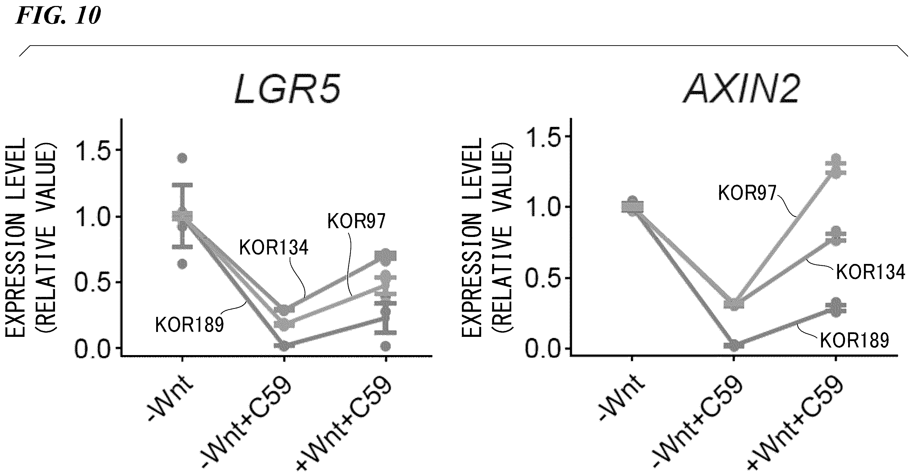

FIG. 10 shows graphs showing results of quantitative RT-PCR measurement of expression levels of Wnt target genes LGR5 and AXIN2 in the NKX2-1-negative lung adenocarcinoma organoid strains (KOR189, KOR134, KOR97) cultured in the absence of Wnt-3A (-Wnt), in the absence of Wnt-3A and in the presence of C59 (-Wnt+C59), and in the presence of Wnt-3A and C59 (+Wnt+C59). In FIG. 10, the relative values of the expression levels of each gene with respect to the expression level of the GAPDH gene are shown as relative values, with the expression level in the absence of Wnt-3A (-Wnt) set to 1.

The results revealed that C59 treatment reduced the expression levels of the Wnt target genes and, in parallel, inhibited the growth of the NKX2-1-negative lung adenocarcinoma organoids. Furthermore, it was revealed that the addition of exogenous Wnt-3A counteracted the effect of the C59 treatment, restoring the expression levels of the Wnt target genes and the growth of the NKX2-1-negative lung adenocarcinoma organoids.

The above-described results indicate that the growth of the NKX2-1-negative lung adenocarcinoma organoids is dependent on their endogenous Wnt ligands.

### [Experimental Example 7]

### (Examination 2 of therapeutic effect of NKX2-1-negative lung adenocarcinoma using Wnt-targetted therapy)

C59 (CAS No. 1243243-89-1) was used to examine the therapeutic effect of NKX2-1-negative lung adenocarcinoma in vivo.

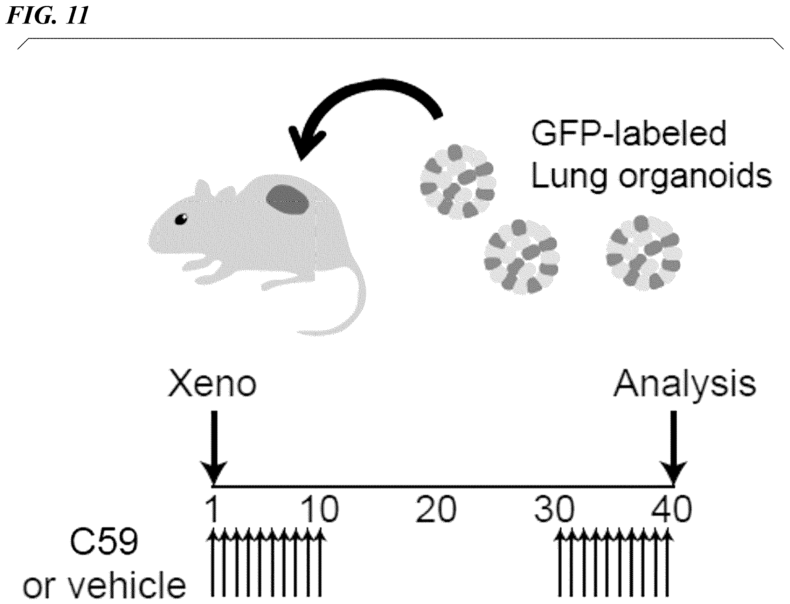

FIG. 11 is a schematic view showing a schedule of this experiment. GFP-labeled lung adenocarcinoma organoids were transplanted under the renal capsule of immunodeficient mice. As the lung adenocarcinoma organoids, Wnt-dependent (NKX2-1 negative) lung adenocarcinoma organoids, KOR80 and KOR97, and Wnt-independent (NKX2-1-positive) lung adenocarcinoma organoids, KOR20 and KOR98, were used.

For 10 days after transplantation and from day 30 to day 40 after transplantation, 50 mg/kg of C59 or vehicle was administered orally twice a day. Subsequently, the mice were euthanized on day 40 after transplantation, and their kidneys were excised and observed. The tumor area was measured as the area of a GFP-positive region.

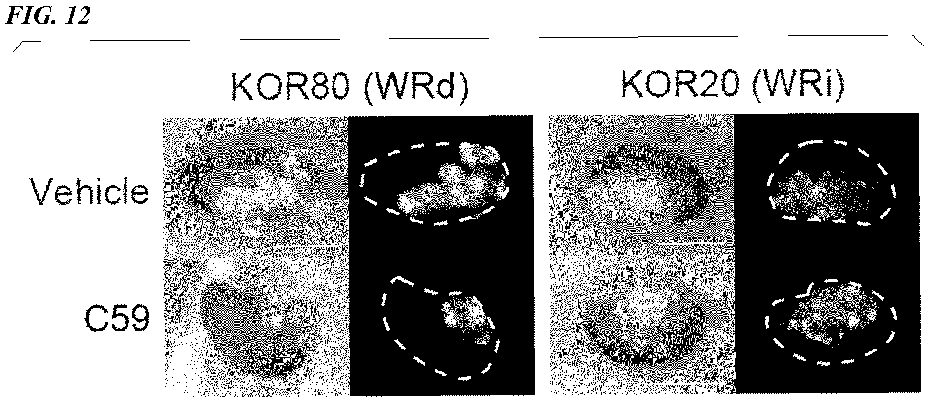

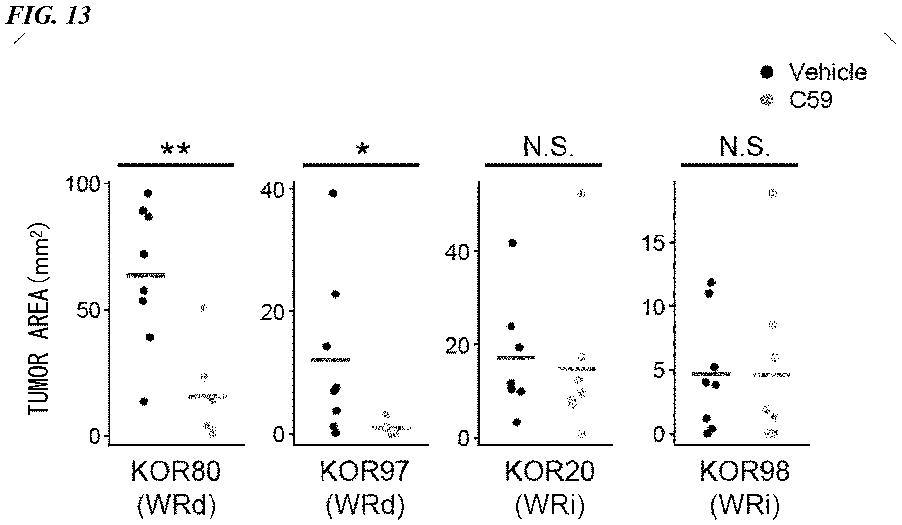

FIG. 12 shows representative photographs of the kidneys excised, and fluorescence micrographs thereof. In addition, FIG. 13 shows graphs showing measurement results of the tumor area in each graft. In FIGS. 12 and 13, "WRd" indicates that it is Wnt-dependent, and "WRi" indicates that it is Wnt-independent. In addition, "vehicle" indicates the result of a control given vehicle, and "C59" indicates the result obtained by administering C59. In addition, in FIG. 13, "*" indicates a significant difference at p < 0.05, "**" indicates a significant difference at p < 0.01, and "N.S." indicates no significant difference.

The results revealed that the administration of C59 significantly reduced the tumor area of Wnt-dependent (NKX2-1 negative) lung adenocarcinoma.

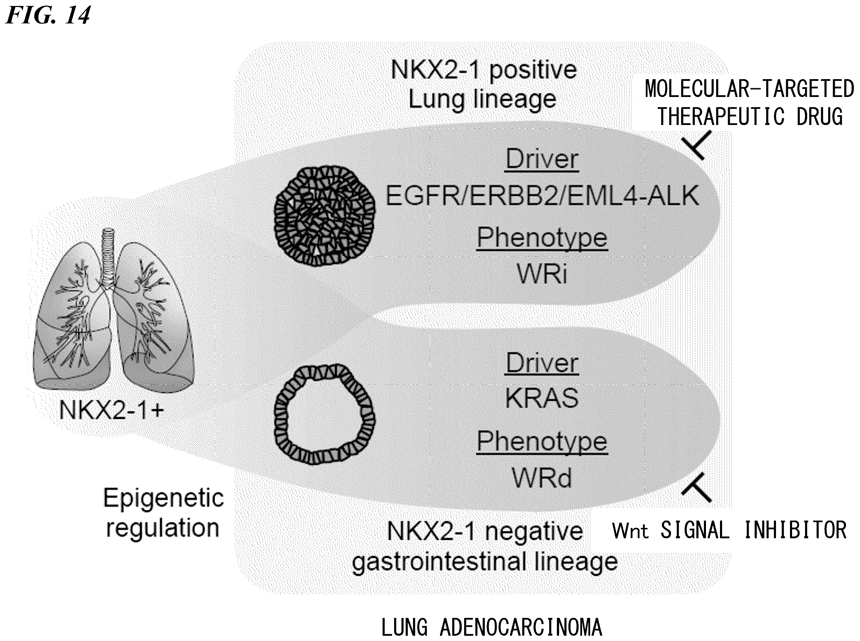

FIG. 14 is a schematic view showing a therapeutic strategy for lung adenocarcinoma. When cancer cells derived from lung adenocarcinoma are NKX2-1 positive, the lung adenocarcinoma is Wnt-independent. In this case, a therapeutic strategy could be considered in which a patient is given a molecular-targeted therapeutic drug selected based on a cancer-causing driver mutation.

In addition, when cancer cells derived from lung adenocarcinoma are NKX2-1 negative, the lung adenocarcinoma is Wnt-dependent. In this case, a therapeutic strategy could be considered in which a patient is given a Porcupine inhibitor.

### INDUSTRIAL APPLICABILITY

According to the present invention, it is possible to provide a novel therapeutic strategy for lung adenocarcinoma, particularly NKX2-1-negative lung adenocarcinoma.

## Claims

1. A therapeutic agent for NK2 homeobox 1 (NKX2-1)-negative lung adenocarcinoma comprising a Porcupine inhibitor as an active component.

2. The therapeutic agent according to claim 1,
wherein the Porcupine inhibitor is C59 (CAS No. 1243243-89-1).

3. An NKX2-1-negative lung adenocarcinoma organoid.

4. A method for screening for a therapeutic agent for NKX2-1-negative lung adenocarcinoma, the method comprising:
a step of culturing an NKX2-1-negative lung adenocarcinoma organoid in the presence of a test substance; and
a step of measuring growth of the NKX2-1-negative lung adenocarcinoma organoid,
wherein a decrease in the growth of the NKX2-1-negative lung adenocarcinoma organoid compared to a case in the absence of the test substance indicates that the test substance is a therapeutic agent for NKX2-1-negative lung adenocarcinoma.
